# EUROPEAN PATENT APPLICATION

(11) **EP 1 126 027 A1**
(43) Date of publication of application: **22.08.2001**
(21) Application number: 01301361.0
(22) Date of filing: 16.02.2001
(51) Int. Cl.: C12N 15/12, C07K 14/47, C12N 9/64, C12N 15/63, C12Q 1/68, G01N 33/68, A61K 38/17

(54) **Cell death inhibition by the caspase-12 activation inhibitor MAGE-3**

(30) Priority: 18.02.2000 JP 2000041927
(71) Applicant: Riken, Wako-shi, Saitama 351-0198 (JP)
(72) Inventor: Morishima, Nobuhiro, Wako-shi, Saitama 351-0198 (JP); Shibata, Takehiko, Wako-shi, Saitama 351-0198 (JP)
(74) Representative: Maschio, Antonio

(57) **Abstract**

The present invention provides a protein which inhibits the activation of caspase-12 and uses of the protein. The present invention relates to a recombinant protein selected from the group consisting of the following (a) and (b):
(a) a protein consisting of the amino acid sequence as shown in SEQ ID NO: 4
(b) a protein which consists of the amino acid sequence as shown in SEQ ID NO: 4 having deletion, substitution or addition of one or several amino acids and which inhibits the activation of caspase-12.

SEQUENCE LISTING

<110> RIKEN

<120> CELL DEATH INHIBITORY PROTEIN

<130> PH-1154

<140>
<141>

<150> JP 2000-41927
<151> 18-FEB-2000

<160> 10

<170> PatentIn Ver. 2.0

<210> 1
<211> 4204
<212> DNA
<213> Homo sapiens

<220>
<221> CDS
<222> (2465)..(3409)

<400> 1
acgcaggcag tgatgtcacc cagaccacac cccttccccc aatgccactt cagggggtac 60

tcagagtcag agacttggtc tgaggggagc agaagcaatc tgcagaggat ggcggtccag 120

gctcagccag gcatcaactt caggaccctg agggatgacc gaaggccccg cccacccacc 180

cccaactccc ccgaccccac caggatctac agcctcagga cccccgtccc aatccttacc 240

ccttgcccca tcaccatctt catgcttacc tccaccccca tccgatcccc atccaggcag 300

aatccagttc cacccctgcc cggaacccag ggtagtaccg ttgccaggat gtgacgccac 360

tgacttgcgc attggaggtc agaagaccgc gagattctcg ccctgagcaa cgagcgacgg 420

cctgacgtcg gcggagggaa gccggcccag gctcggtgag gaggcaaggt aagacgctga 480

gggaggactg aggcgggcct cacctcagac agagggcctc aaataatcca gtgctgcctc 540

tgctgccggg cctgggccac cccgcagggg aagacttcca ggctgggtcg ccactacctc 600

accccgccga cccccgccgc tttagccacg gggaactctg gggacagagc ttaatgtggc 660

cagggcaggg ctggttagaa gaggtcaggg cccacgctgt ggcaggaatc aaggtcagga 720

ccccgagagg gaactgaggg cagcctaacc accaccctca ccaccattcc cgtcccccaa 780

cacccaaccc cacccccatc ccccattccc atccccaccc ccacccctat cctggcagaa 840

tccgggcttt gcccctggta tcaagtcacg gaagctccgg gaatggcggc caggcacgtg 900

agtcctgagg ttcacatcta cggctaaggg agggaagggg ttcggtatcg cgagtatggc 960

cgttgggagg cagcgaaagg gcccaggcct cctggaagac agtggagtcc tgaggggacc 1020

cagcatgcca ggacaggggg cccactgtac ccctgtctca aaccgaggca ccttttcatt 1080

cggctacggg aatcctaggg atgcagaccc acttcagcag ggggttgggg cccagccctg 1140

cgaggagtca tggggaggaa gaagagggag gactgagggg accttggagt ccagatcagt 1200

ggcaaccttg ggctggggga tgctgggcac agtggccaaa tgtgctctgt gctcattgcg 1260

ccttcagggt gaccagagag ttgagggctg tggtctgaag agtgggactt caggtcagca 1320

gagggaggaa tcccaggatc tgcagggccc aaggtgtacc cccaaggggc ccctatgtgg 1380

tggacagatg cagtggtcct aggatctgcc aagcatccag gtgaagagac tgagggagga 1440

ttgagggtac ccctgggaca gaatgcggac tgggggcccc ataaaaatct gccctgctcc 1500

tgctgttacc tcagagagcc tgggcagggc tgtcagctga ggtccctcca ttatcctagg 1560

atcactgatg tcagggaagg ggaagccttg gtctgagggg gctgcactca gggcagtaga 1620

gggaggctct cagaccctac taggagtgga ggtgaggacc aagcagtctc ctcacccagg 1680

gtacatggac ttcaataaat ttggacatct ctcgttgtcc tttccgggag gacctgggaa 1740

tgtatggcca gatgtgggtc ccctcatgtt tttctgtacc atatcaggta tgtgagttct 1800

tgacatgaga gattctcagg ccagcagaag ggagggatta ggccctataa ggagaaaggt 1860

gagggccctg agtgagcaca gaggggatcc tccaccccag tagagtgggg acctcacaga 1920

gtctggccaa ccctcctgac agttctggga atccgtggct gcgtttgctg tctgcacatt 1980

gggggcccgt ggattcctct cccaggaatc aggagctcca ggaacaaggc agtgaggact 2040

tggtctgagg cagtgtcctc aggtcacaga gtagaggggg ctcagatagt gccaacggtg 2100

aaggtttgcc ttggattcaa accaagggcc ccacctgccc cagaacacat ggactccaga 2160

gcgcctggcc tcaccctcaa tactttcagt cctgcagcct cagcatgcgc tggccggatg 2220

taccctgagg tgccctctca cttcctcctt caggttctga ggggacaggc tgacctggag 2280

gaccagaggc ccccggagga gcactgaagg agaagatctg taagtaagcc tttgttagag 2340

cctccaaggt tccattcagt actcagctga ggtctctcac atgctccctc tctccccagg 2400

ccagtgggtc tccattgccc agctcctgcc cacactcccg cctgttgccc tgaccagagt 2460

catc atg cct ctt gag cag agg agt cag cac tgc aag cct gaa gaa ggc 2509
Met Pro Leu Glu Gln Arg Ser Gln His Cys Lys Pro Glu Glu Gly
1               5                   10                  15

ctt gag gcc cga gga gag gcc ctg ggc ctg gtg ggt gcg cag gct cct 2557
Leu Glu Ala Arg Gly Glu Ala Leu Gly Leu Val Gly Ala Gln Ala Pro

                20                  25                  30

gct act gag gag cag gag gct gcc tcc tcc tct tct act cta gtt gaa 2605
Ala Thr Glu Glu Gln Glu Ala Ala Ser Ser Ser Ser Thr Leu Val Glu
            35                  40                  45

gtc acc ctg ggg gag gtg cct gct gcc gag tca cca gat cct ccc cag 2653
Val Thr Leu Gly Glu Val Pro Ala Ala Glu Ser Pro Asp Pro Pro Gln
        50                  55                  60

agt cct cag gga gcc tcc agc ctc ccc act acc atg aac tac cct ctc 2701
Ser Pro Gln Gly Ala Ser Ser Leu Pro Thr Thr Met Asn Tyr Pro Leu
    65                  70                  75

tgg agc caa tcc tat gag gac tcc agc aac caa gaa gag gag ggg cca 2749
Trp Ser Gln Ser Tyr Glu Asp Ser Ser Asn Gln Glu Glu Glu Gly Pro
80                  85                  90                  95

agc acc ttc cct gac ctg gag tcc gag ttc caa gca gca ctc agt agg 2797
Ser Thr Phe Pro Asp Leu Glu Ser Glu Phe Gln Ala Ala Leu Ser Arg
                100                 105                 110

aag gtg gcc gag ttg gtt cat ttt ctg ctc ctc aag tat cga gcc agg 2845
Lys Val Ala Glu Leu Val His Phe Leu Leu Leu Lys Tyr Arg Ala Arg
            115                 120                 125

gag ccg gtc aca aag gca gaa atg ctg ggg agt gtc gtc gga aat tgg 2893
Glu Pro Val Thr Lys Ala Glu Met Leu Gly Ser Val Val Gly Asn Trp
        130                 135                 140

cag tat ttc ttt cct gtg atc ttc agc aaa gct tcc agt tcc ttg cag 2941
Gln Tyr Phe Phe Pro Val Ile Phe Ser Lys Ala Ser Ser Ser Leu Gln
    145                 150                 155

ctg gtc ttt ggc atc gag ctg atg gaa gtg gac ccc atc ggc cac ttg 2989
Leu Val Phe Gly Ile Glu Leu Met Glu Val Asp Pro Ile Gly His Leu
160                 165                 170                 175

tac atc ttt gcc acc tgc ctg ggc ctc tcc tac gat ggc ctg ctg ggt 3037
Tyr Ile Phe Ala Thr Cys Leu Gly Leu Ser Tyr Asp Gly Leu Leu Gly
                180                 185                 190

gac aat cag atc atg ccc aag gca ggc ctc ctg ata atc gtc ctg gcc 3085
Asp Asn Gln Ile Met Pro Lys Ala Gly Leu Leu Ile Ile Val Leu Ala
            195                 200                 205

ata atc gca aga gag ggc gac tgt gcc cct gag gag aaa atc tgg gag 3133
Ile Ile Ala Arg Glu Gly Asp Cys Ala Pro Glu Glu Lys Ile Trp Glu
        210                 215                 220

gag ctg agt gtg tta gag gtg ttt gag ggg agg gaa gac agt atc ttg 3181
Glu Leu Ser Val Leu Glu Val Phe Glu Gly Arg Glu Asp Ser Ile Leu
    225                 230                 235

ggg gat ccc aag aag ctg ctc acc caa cat ttc gtg cag gaa aac tac 3229
Gly Asp Pro Lys Lys Leu Leu Thr Gln His Phe Val Gln Glu Asn Tyr
240                 245                 250                 255

ctg gag tac cgg cag gtc ccc ggc agt gat cct gca tgt tat gaa ttc 3277
Leu Glu Tyr Arg Gln Val Pro Gly Ser Asp Pro Ala Cys Tyr Glu Phe
                260                 265                 270

ctg tgg ggt cca agg gcc ctc gtt gaa acc agc tat gtg aaa gtc ctg 3325
Leu Trp Gly Pro Arg Ala Leu Val Glu Thr Ser Tyr Val Lys Val Leu
            275                 280                 285

cac cat atg gta aag atc agt gga gga cct cac att tcc tac cca ccc 3373
His His Met Val Lys Ile Ser Gly Gly Pro His Ile Ser Tyr Pro Pro
        290                 295                 300

ctg cat gag tgg gtt ttg aga gag ggg gaa gag tga gtctgagcac 3419
Leu His Glu Trp Val Leu Arg Glu Gly Glu Glu
305 310 315

gagttgcagc cagggccagt gggagggggt ctgggccagt gcaccttccg gggccgcatc 3479

ccttagtttc cactgcctcc tgtgacgtga ggcccattct tcactctttg aagcgagcag 3539

tcagcattct tagtagtggg tttctgttct gttggatgac tttgagatta ttctttgttt 3599

cctgttggag ttgttcaaat gttcctttta acggatggtt gaatgagcgt cagcatccag 3659

gtttatgaat gacagtagtc acacatagtg ctgtttatat agtttaggag taagagtctt 3719

gttttttact caaattggga aatccattcc attttgtgaa ttgtgacata ataatagcag 3779

tggtaaaagt atttgcttaa aattgtgagc gaattagcaa taacatacat gagataactc 3839

aagaaatcaa aagatagttg attcttgcct tgtacctcaa tctattctgt aaaattaaac 3899

aaatatgcaa accaggattt ccttgacttc tttgagaatg caagcgaaat taaatctgaa 3959

taaataattc ttcctcttca ctggctcgtt tcttttccgt tcactcagca tctgctctgt 4019

gggaggccct gggttagtag tggggatgct aaggtaagcc agactcacgc ctacccatag 4079

ggctgtagag cctaggacct gcagtcatat aattaaggtg gtgagaagtc ctgtaagatg 4139

tagaggaaat gtaagagagg ggtgagggtg tggcgctccg ggtgagagta gtggagtgtc 4199

agtgc 4204

<210> 2

<211> 314

<212> PRT

<213> Homo sapiens

<400> 2

Met Pro Leu Glu Gln Arg Ser Gln His Cys Lys Pro Glu Glu Gly Leu
1               5                   10                  15

Glu Ala Arg Gly Glu Ala Leu Gly Leu Val Gly Ala Gln Ala Pro Ala
            20                  25                  30

Thr Glu Glu Gln Glu Ala Ala Ser Ser Ser Ser Thr Leu Val Glu Val

        35                  40                  45

Thr Leu Gly Glu Val Pro Ala Ala Glu Ser Pro Asp Pro Pro Gln Ser
    50                  55                  60

Pro Gln Gly Ala Ser Ser Leu Pro Thr Thr Met Asn Tyr Pro Leu Trp
65                  70                  75                  80

Ser Gln Ser Tyr Glu Asp Ser Ser Asn Gln Glu Glu Glu Gly Pro Ser
                85                  90                  95

Thr Phe Pro Asp Leu Glu Ser Glu Phe Gln Ala Ala Leu Ser Arg Lys
            100                 105                 110

Val Ala Glu Leu Val His Phe Leu Leu Leu Lys Tyr Arg Ala Arg Glu
        115                 120                 125

Pro Val Thr Lys Ala Glu Met Leu Gly Ser Val Val Gly Asn Trp Gln
    130                 135                 140

Tyr Phe Phe Pro Val Ile Phe Ser Lys Ala Ser Ser Ser Leu Gln Leu
145                 150                 155                 160

Val Phe Gly Ile Glu Leu Met Glu Val Asp Pro Ile Gly His Leu Tyr
                165                 170                 175

Ile Phe Ala Thr Cys Leu Gly Leu Ser Tyr Asp Gly Leu Leu Gly Asp
            180                 185                 190

Asn Gln Ile Met Pro Lys Ala Gly Leu Leu Ile Ile Val Leu Ala Ile
        195                 200                 205
Ile Ala Arg Glu Gly Asp Cys Ala Pro Glu Glu Lys Ile Trp Glu Glu
    210                 215                 220
Leu Ser Val Leu Glu Val Phe Glu Gly Arg Glu Asp Ser Ile Leu Gly
225                 230                 235                 240
Asp Pro Lys Lys Leu Leu Thr Gln His Phe Val Gln Glu Asn Tyr Leu
                245                 250                 255
Glu Tyr Arg Gln Val Pro Gly Ser Asp Pro Ala Cys Tyr Glu Phe Leu
            260                 265                 270
Trp Gly Pro Arg Ala Leu Val Glu Thr Ser Tyr Val Lys Val Leu His
        275                 280                 285
His Met Val Lys Ile Ser Gly Gly Pro His Ile Ser Tyr Pro Pro Leu
    290                 295                 300
His Glu Trp Val Leu Arg Glu Gly Glu Glu
305                 310

<210> 3
<211> 666
<212> DNA
<213> Homo sapiens

<220>
<221> CDS
<222> (1)..(666)

<400> 3
ggg cca agc acc ttc cct gac ctg gag tcc gag ttc caa gca gca ctc 48
Gly Pro Ser Thr Phe Pro Asp Leu Glu Ser Glu Phe Gln Ala Ala Leu
1               5                   10                  15

agt agg aag gtg gcc gag ttg gtt cat ttt ctg ctc ctc aag tat cga 96
Ser Arg Lys Val Ala Glu Leu Val His Phe Leu Leu Leu Lys Tyr Arg
            20                  25                  30

gcc agg gag ccg gtc aca aag gca gaa atg ctg ggg agt gtc gtc gga 144
Ala Arg Glu Pro Val Thr Lys Ala Glu Met Leu Gly Ser Val Val Gly
        35                  40                  45

aat tgg cag tat ttc ttt cct gtg atc ttc agc aaa gct tcc agt tcc 192
Asn Trp Gln Tyr Phe Phe Pro Val Ile Phe Ser Lys Ala Ser Ser Ser
    50                  55                  60

ttg cag ctg gtc ttt ggc atc gag ctg atg gaa gtg gac ccc atc ggc 240
Leu Gln Leu Val Phe Gly Ile Glu Leu Met Glu Val Asp Pro Ile Gly
65                  70                  75                  80

cac ttg tac atc ttt gcc acc tgc ctg ggc ctc tcc tac gat ggc ctg 288
His Leu Tyr Ile Phe Ala Thr Cys Leu Gly Leu Ser Tyr Asp Gly Leu
                85                  90                  95

ctg ggt gac aat cag atc atg ccc aag gca ggc ctc ctg ata atc gtc 336
Leu Gly Asp Asn Gln Ile Met Pro Lys Ala Gly Leu Leu Ile Ile Val
            100                 105                 110

ctg gcc ata atc gca aga gag ggc gac tgt gcc cct gag gag aaa atc 384
Leu Ala Ile Ile Ala Arg Glu Gly Asp Cys Ala Pro Glu Glu Lys Ile
        115                 120                 125

tgg gag gag ctg agt gtg tta gag gtg ttt gag ggg agg gaa gac agt 432
Trp Glu Glu Leu Ser Val Leu Glu Val Phe Glu Gly Arg Glu Asp Ser
    130                 135                 140

atc ttg ggg gat ccc aag aag ctg ctc acc caa cat ttc gtg cag gaa 480
Ile Leu Gly Asp Pro Lys Lys Leu Leu Thr Gln His Phe Val Gln Glu
145                 150                 155                 160

aac tac ctg gag tac cgg cag gtc ccc ggc agt gat cct gca tgt tat 528
Asn Tyr Leu Glu Tyr Arg Gln Val Pro Gly Ser Asp Pro Ala Cys Tyr
                165                 170                 175

gaa ttc ctg tgg ggt cca agg gcc ctc gtt gaa acc agc tat gtg aaa 576
Glu Phe Leu Trp Gly Pro Arg Ala Leu Val Glu Thr Ser Tyr Val Lys
            180                 185                 190

gtc ctg cac cat atg gta aag atc agt gga gga cct cac att tcc tac 624
Val Leu His His Met Val Lys Ile Ser Gly Gly Pro His Ile Ser Tyr
        195                 200                 205

cca ccc ctg cat gag tgg gtt ttg aga gag ggg gaa gag tga 666
Pro Pro Leu His Glu Trp Val Leu Arg Glu Gly Glu Glu
    210                 215                 220

<210> 4
<211> 221
<212> PRT
<213> Homo sapiens

<400> 4
Gly Pro Ser Thr Phe Pro Asp Leu Glu Ser Glu Phe Gln Ala Ala Leu
1               5                   10                  15

Ser Arg Lys Val Ala Glu Leu Val His Phe Leu Leu Leu Lys Tyr Arg
            20                  25                  30

Ala Arg Glu Pro Val Thr Lys Ala Glu Met Leu Gly Ser Val Val Gly
        35                  40                  45

Asn Trp Gln Tyr Phe Phe Pro Val Ile Phe Ser Lys Ala Ser Ser Ser
    50                  55                  60

Leu Gln Leu Val Phe Gly Ile Glu Leu Met Glu Val Asp Pro Ile Gly
65                  70                  75                  80

His Leu Tyr Ile Phe Ala Thr Cys Leu Gly Leu Ser Tyr Asp Gly Leu
                85                  90                  95

Leu Gly Asp Asn Gln Ile Met Pro Lys Ala Gly Leu Leu Ile Ile Val
            100                 105                 110

Leu Ala Ile Ile Ala Arg Glu Gly Asp Cys Ala Pro Glu Glu Lys Ile
        115                 120                 125

Trp Glu Glu Leu Ser Val Leu Glu Val Phe Glu Gly Arg Glu Asp Ser
    130                 135                 140

Ile Leu Gly Asp Pro Lys Lys Leu Leu Thr Gln His Phe Val Gln Glu
145                 150                 155                 160

Asn Tyr Leu Glu Tyr Arg Gln Val Pro Gly Ser Asp Pro Ala Cys Tyr
                165                 170                 175

Glu Phe Leu Trp Gly Pro Arg Ala Leu Val Glu Thr Ser Tyr Val Lys
            180                 185                 190

Val Leu His His Met Val Lys Ile Ser Gly Gly Pro His Ile Ser Tyr
        195                 200                 205

Pro Pro Leu His Glu Trp Val Leu Arg Glu Gly Glu Glu
    210                 215                 220

<210> 5
<211> 20
<212> DNA
<213> Artificial Sequence

<220>
<223> Description of Artificial Sequence:synthetic DNA

<400> 5
gcccagctcc tgcccacact                                                    20

<210> 6
<211> 19
<212> DNA
<213> Artificial Sequence

<220>
<223> Description of Artificial Sequence:synthetic DNA

<400> 6
ggatgcggcc ccggaaggt                                                     19

<210> 7
<211> 32
<212> DNA
<213> Artificial Sequence

<220>
<223> Description of Artificial Sequence:synthetic DNA

<400> 7

ctcgatatcg caccatgcct cttgagcaga gg                                    32

<210> 8
<211> 27
<212> DNA
<213> Artificial Sequence

<220>
<223> Description of Artificial Sequence:synthetic DNA

<400> 8
ctcctcgagt cactcttccc cctctct                                          27

<210> 9
<211> 14
<212> PRT
<213> Artificial Sequence

<220>
<223> Description of Artificial Sequence:synthetic peptide

<400> 9
Met Arg Gly Ser His His His His His His Gly Met Ala Ser
  1               5                   10

<210> 10
<211> 18

<212> PRT
<213> Artificial Sequence
<220>
<223> Description of Artificial Sequence:synthetic peptide
<400> 10

## Description

### BACKGROUND OF THE INVENTION

### 1. Field of the Invention

The present invention relates to a protein which inhibits the activation of caspase-12 by binding to caspase-12 or the precursor thereof, and a cell death inhibitor comprising the protein.

### 2. Description of the Related Art

Caspases are a family of proteases which play key roles in the apoptosis of multicellular organisms. Fourteen members of the caspase family have so far been identified from human and mouse (Thornberry, N.A. & Lazebnik, Y., "Caspases, enemies within", Science 281, 1312-1316 (1998)). The functions of caspases are achieved by cleaving a group of specific proteins with their specific proteolysis activity. It is believed that one of the reasons why a plurality of caspases exist is because different sets of caspases function in response to a variety of apoptotic stimuli.

It is now being elucidated that, while caspases are involved in normal functions such as, e.g., morphogenesis in the developmental process, the maintenance of homeostasis in the adult, and the removal of cells harmful to the body, they may cause severe illness such as neurodegenerative diseases if they should be activated abnormally (Yuan, J. & Yankner, B.A., Nat. Cell Biol. 1, E44-45 (1999)).

Caspases are biosynthesized as inactive precursors and activated through intramolecular specific cleavage (processing). Thus, it may be possible to inhibit caspase-induced apoptosis if this processing can be inhibited.

### OBJECTS AND SUMMARY OF THE INVENTION

It is an object of the invention to provide a protein which inhibits the activation of caspase-12 and a cell death inhibitor comprising the protein.

As a result of extensive and intensive researches toward the solution of the above problem, the present inventor has found that a cancer-specific protein MAGE-3 or a truncated form thereof specifically binds to caspase-12 or the precursor thereof (hereinafter, frequently referred to as "pro-caspase-12"). Thus, the present invention has been achieved.

The present invention relates to a recombinant protein selected from the group consisting of the following (a) and (b), or a gene coding for the recombinant protein:
(a) a protein consisting of the amino acid sequence as shown in SEQ ID NO: 4
(b) a protein which consists of the amino acid sequence as shown in SEQ ID NO: 4 having deletion, substitution or addition of one or several amino acids and which inhibits the activation of caspase-12.

In one embodiment of the invention, the above gene is a gene consisting of a DNA selected from the group consisting of the following (c) and (d):
(c) a DNA consisting of the nucleotide sequence as shown in SEQ ID NO: 3
(d) a DNA which hybridizes to the nucleotide sequence as shown in SEQ ID NO: 3 under stringent conditions and which codes for a protein that inhibits the activation of caspase-12.

Further, the present invention relates to a recombinant vector comprising the above gene.

Further, the present invention relates to a transformant comprising the above recombinant vector.

Further, the present invention relates to a method of producing a protein which inhibits the activation of caspase-12, comprising culturing the above transformant in a medium and recovering the protein from the resultant culture.

Further, the present invention relates to a complex formed by the binding of a MAGE-3 protein and/or a truncated form thereof to caspase-12 or the precursor thereof.

Further, the present invention relates to a cell death inhibitor comprising a MAGE-3 protein and/or a truncated form thereof as an active ingredient.

In one embodiment of the invention, the MAGE-3 protein is a recombinant protein selected from the group consisting of the following (e) and (f):
(e) a protein consisting of the amino acid sequence as shown in SEQ ID NO: 2
(f) a protein which consists of the amino acid sequence as shown in SEQ ID NO: 2 having deletion, substitution or addition of one or several amino acids and which inhibits the activation of caspase-12. In one embodiment of the invention, the truncated
form of a MAGE-3 protein is a recombinant protein selected from the group consisting
of (a) and (b) described above.

In one embodiment of the invention, the cell death is at least one selected from the group consisting of apoptosis, necrosis, scheduled cell death, programmed cell death and cell injury.

Further, the present invention relates to a therapeutic agent for cell death related diseases, comprising a MAGE-3 protein and/or a truncated form thereof as an active ingredient.

In one embodiment of the invention, the cell death related disease is at least one selected from the group consisting of Alzheimer's disease, neurodegenerative diseases, autoimmune diseases, amyotrophy and organ disorders.

Further, the present invention relates to a method of inhibiting the activation of caspase-12, comprising binding a MAGE-3 protein or a truncated form thereof to caspase-12 or the precursor thereof.

Further, the present invention relates to a method of detecting anti-apoptotic activity in a tissue or cell, comprising treating said tissue or cell with an antibody which specifically recognizes a MAGE-3 protein or a truncated form thereof and detecting expression of said MAGE-3 protein or said truncated form in said tissue or cell, wherein detection of a high expression of said MAGE-3 protein or said truncated form thereof indicates that said tissue of cell has anti-apoptotic activity.

Further, the present invention relates to a method of detecting anti-apoptotic activity in a tissue or cell, comprising detecting the expression of an mRNA encoding a MAGE-3 protein or a truncated form thereof in said tissue or cell.

### BRIEF DESCRIPTION OF THE DRAWINGS

Fig. 1 shows the SDS-polyacrylamide gel electrophoresis pattern of a MAGE-3 protein purified from *Escherichia coli.*

Fig. 2 shows the results of a test examining the binding of MAGE-3 to caspase-12 in cultured mammalian cells.

Fig. 3 shows the results of a test examining the binding of MAGE-3 to p10 region of caspase-12.

Fig. 4 shows the amino acid sequence of MAGE-3.

Fig. 5 shows the results of comparison of the binding affinities between MAGE-3 and two forms of caspase-12 (i.e. mature enzyme and proenzyme).

Fig.6 shows a schematic drawing of pro-caspase-12.

Fig. 7 shows the results of a test examining the inhibition of pro-caspase-12 activation by MAGE-3.

Fig. 8 shows the results of a test inducing in cells resistance to apoptosis by high expression of MAGE-3.

### DETAILED DESCRIPTION OF THE INVENTION

The present specification includes the contents of the specifications and/or drawings of the Japanese Patent Applications No. 2000-41927 based on which the present application claims priority.

The present invention relates to a cancer-specific protein designated "melanoma associated antigen 3" (MAGE-3), truncated forms thereof, and their uses. The present invention has been achieved based on the finding that the above protein has a function to inhibit the processing of caspase-12.

Pro-caspase-12 is localized to the endoplasmic reticulum (ER) in cells, and matures into active caspase-12 through autoprocessing triggered by ER-specific stress (Nakagawa, T. et al., Nature 403, 98-103 (2000)). It has been suggested that ER stress is one of the causes of Alzheimer's disease, a human neurodegenerative disease. Accordingly, by inhibiting the activation from pro-caspase-12 to mature caspase-12 or the activation of mature caspase-12 itself using MAGE-3, ER stress-related diseases such as Alzheimer's disease can be prevented or treated. Furthermore, since the inhibition of caspase activation by MAGE-3 protein is specific to caspase-12, an inhibitor using MAGE-3 is highly specific and thus expected to give no adverse effect upon normal functions of other caspases.

The expression "inhibit the activation of caspase-12" used herein means to inhibit the processing of pro-caspase-12 to mature caspase-12 by specifically binding to pro-caspase-12, or to inhibit the function of mature (active) caspase-12 itself by specifically binding to mature caspase-12. As a result of such inhibition, it becomes possible to inhibit cell death caused by mature caspase-12. In the present invention, however, it is preferable to allow MAGE-3 or truncated forms thereof to bind to pro-caspase-12.

In the present invention, it is possible to inhibit the activation of caspase-12 using MAGE-3 (Gaugler, B. et al., J. Exp. Med. 179, 921-930 (1994)) protein or truncated forms thereof. In the present invention, MAGE-3 protein is expressed as "MAGE-3" and a gene encoding MAGE-3 protein is expressed as *MAGE-3*. MAGE-3 and truncated forms thereof are specific to caspase-12 and do not exhibit binding activity to other caspases.

In the present invention, *MAGE-3* is isolated by conventional genetic engineering techniques. The resultant gene is introduced into a vector to prepare a recombinant vector, which is then introduced into an appropriate host to create a transformant. When the recombinant vector is constructed as an expression vector that is capable of functioning in the host, MAGE-3 can be obtained by culturing the transformant.

### 1. Cloning of Genes Encoding MAGE-3 and Truncated Forms Thereof

First, *MAGE-3* is cloned in order to obtain the MAGE-3 of the invention. Although the nucleotide sequence of *MAGE-3* is known (Gaugler, B. et al., J. Exp. Med. 179, 921-930 (1994)), it may also be prepared as described below by genetic engineering techniques.

### (1) Preparation and Screening of MAGE-3 cDNA Library

Preparation of the mRNA of *MAGE-3* may be performed by conventional techniques. For example, a tissue or cells from the testis or tumor are treated with guanidine reagent or phenol reagent to obtain total RNA. Then, poly(A⁺) RNA (mRNA) is obtained therefrom by the affinity column method using oligo(dT)-cellulose or poly U-Sepharose using Sepharose 2B as a carrier, or by the batch method. With the resultant mRNA as a template, a single-stranded cDNA is synthesized using oligo(dT) primers and a reverse transcriptase. Then, a double-stranded cDNA is synthesized from the single-stranded cDNA. The thus obtained double-stranded cDNA is integrated into an appropriate cloning vector to prepare a recombinant vector. A host such as E. *coli* is transformed with the recombinant vector. The resultant transformants are selected using tetracycline resistance and ampicillin resistance as indicators to thereby obtain a cDNA library.

Alternatively, a commercial cDNA library (e.g., a human testis cDNA library; Clontech) may be used in the present invention.

As a screening method to select those clones having the DNA of interest from the selected transformants, the immunoscreening technique using antibodies or a method in which primers are synthesized based on the known sequence of the DNA followed by PCR using the primers may be use, for example,

### (2) Preparation of Truncated MAGE-3

In the present invention, genes encoding truncated forms of MAGE-3 (hereinafter, referred to as "truncated *MAGE-3*") can be designed and synthesized. A truncated form of MAGE-3 means a MAGE-3 protein which has a deletion of amino acids at the N-terminus or C-terminus or both ends of the full-length amino acid sequence of MAGE-3 (SEQ ID NO: 2), the total number of the amino acids deleted being 312 at the maximum. More specifically, a truncated form of MAGE-3 means a polypeptide or protein which has at least 2, preferably 10 or more amino acids of SEQ ID NO: 2 remaining after deletion. Specific examples of the truncated form of MAGE-3 of the invention include a MAGE-3 protein lacking amino acids from position 1 to position 81 (Ser) in SEQ ID NO: 2; a MAGE-3 protein lacking amino acids from position 1 to position 88 (Ser) in SEQ ID NO: 2; a MAGE-3 protein lacking amino acids from position 1 to position 90 (Gln) in SEQ ID NO: 2; and a MAGE-3 protein lacking amino acids from position 1 to position 93 (Glu) in SEQ ID NO: 2.

Of the truncated forms of MAGE-3 protein of the invention (hereinafter, referred to as "truncated MAGE-3"), one lacking amino acids from position 1 to position 93 in SEQ ID NO: 2 is represented by SEQ ID NO: 4. This amino acid sequence corresponds to a partial amino acid sequence of SEQ ID NO: 2 spanning from position 94 to position 314.

These truncated proteins can be obtained by performing PCR using *MAGE-3* (SEQ ID NO: 1; Gaugler, B, et al., J. Exp. Med. 179, 921-930 (1994)) as a template and using primers designed based on partial sequences which are located in any region of SEQ ID NO: 1 outside of the coding region of the truncated protein of interest. It should be noted that it is desirable to add a nucleotide sequence containing a translation start codon to the 5' end of the coding region in order to synthesize truncated MAGE-3.

### (3) Creation of Mutants of MAGE-3 and Mutants of Truncated MAGE-3

In the present invention, it is possible to introduce a mutation(s) into a part of the amino acid sequence of MAGE-3 or truncated MAGE-3. Therefore, these mutated proteins are also included in the MAGE-3 or truncated MAGE-3 of the invention. For the introduction of a mutation into an amino acid, a technique is used in which a mutation is introduced into the nucleotide sequence of a gene encoding the target amino acid.

The introduction of a mutation into a gene may be performed by known techniques such as the method of Kunkel or the gapped duplex method, or by techniques based on these methods. For example, a mutation may be introduced by site-specific mutagenesis using a mutation-introduced oligonucleotide as a primer (Yoshikawa, F. et al., J. Biol. Chem. 271: 18277-18284 (1996)). This may be carried out using a commercial mutagenesis kit such as Mutan-K (Takara), Mutan-G (Takara) or LA PCR in vitro Mutagenesis Series kit (Takara).

For example, a primer is synthesized which consists of a mutated nucleotide and flanking regions thereof (about 10 nucleotides for each) that are found in the nucleotide sequence of *MAGE-3* or truncated *MAGE-3.* Then, PCR is performed with *MAGE-3* as a template and using the above primer. The PCR product is purified and treated with an appropriate restriction enzyme(s) to thereby obtain *MAGE-3* or truncated *MAGE-3* of interest.

### (4) Determination of Nucleotide Sequences

The nucleotide sequence of the thus obtained gene is determined. This sequencing may be performed by the chemical modification method of Maxam-Gilbert or the dideoxynucleotide chain termination method using M13 phage. However, sequencing is usually performed with an automated DNA sequencer (e.g., 377A DNA Sequencer; Perkin-Elmer).

The nucleotide sequence of *MAGE-3* is shown in SEQ ID NO: 1 and the amino acid sequence of MAGE-3 in SEQ ID NO: 2. The nucleotide sequence of a truncated *MAGE-3* is shown in SEQ ID NO: 3 and the amino acid sequence of this truncated MAGE-3 in SEQ ID NO: 4. As long as the polypeptides consisting of these amino acid sequences have specific binding activity to caspase-12 or pro-caspase-12, (i.e., as long as they can inhibit the activation of caspase-12), those amino acid sequences may have mutations such as deletion, substitution or addition of one or more (e.g., one or several, preferably about one to ten, more preferably one to five) amino acids.

In addition to the gene (SEQ ID NO: 3) encoding the amino acids included in the truncated MAGE-3 of the invention (SEQ ID NO: 4), degenerate isomers encoding the same polypeptide which differ only in degenerate codons are also included in the gene of the invention. Furthermore, DNAs which hybridize to the nucleotide sequence of the truncated *MAGE-3* (SEQ ID NO: 3) under stringent conditions and which encode a protein that inhibits the activation of caspase-12 are included in the gene of the invention. The "stringent conditions" used herein means such conditions under which the so-called specific hybrids are formed, but non-specific hybrids are not formed. For example, those conditions may be given under which two highly homologous nucleic acids (i.e., DNAs having 60% or more, preferably 80% or more homology to each other) hybridize to each other, but hybridization does not occur between two nucleic acids with less homology. More specifically, a sodium concentration of 15-900 mM, preferably 15-150 mM, and a temperature of 37-70°C, preferably 68°C, are used.

Once the nucleotide sequence of the truncated *MAGE-3* of the invention has been determined, the gene of the invention can be obtained by chemical synthesis or by PCR using primers synthesized based on the determined nucleotide sequence.

### 3. Preparation of Recombinant Vectors and Transformants Comprising the Gene of the Invention

### (1) Preparation of Recombinant Vectors

The recombinant vector of the invention can be obtained by ligating (inserting) the gene of the invention into an appropriate vector. The vector into which the gene of the invention is to be inserted is not particularly limited as long as it is replicable in a host. For example, plasmid DNA, phage DNA or the like may be used.

Specific examples of plasmid DNA include a commercial plasmid such as pBluescript SK+ (Stratagene). Other examples of plasmids which may be used in the invention include *E*. *coli*-derived plasmids (e.g., pBR322, pBR325, pUC118 and pUC119), *Bacillus subtilis*-derived plasmids (e.g., pUB110 and pTP5) and yeast-derived plasmids (e.g., YEp13, YEp24 and YCp50). Specific examples of phage DNA include λ phages (e.g., Charon4A, Charon21A, EMBL3, EMBL4, λ gt10, λ gt11 and λ ZAP). Further, an animal virus vector such as retrovirus, adenovirus or vaccinia virus; or an insect virus vector such as baculovirus may also be used. Further, a fusion plasmid in which a gene expression activating protein (such as B42) is ligated (e.g., pJG4-5) may be used. A fusion plasmid which may be used in the invention is not limited to the above-mentioned pJG4-5. A fusion plasmid in which GST, GFP, His-tag, Myc-tag or the like is ligated may also be used in the invention.

For insertion of the gene of the invention into a vector, a method may be employed in which the purified DNA is digested with an appropriate restriction enzyme and then inserted into the restriction site or multi-cloning site of an appropriate vector DNA for ligation to the vector.

The gene of the invention must be operably linked to the vector, For this purpose, the vector of the invention may contain, if desired, cis elements such as an enhancer, a splicing signal, a poly(A) addition signal, selection markers, a ribosome binding sequence (SD sequence) or the like in addition to a promoter and the gene of the invention. As the selection marker, chloramphenicol resistance gene, ampicillin resistance gene, dihydrofolate reductase gene, neomycin resistance gene, or the like may be enumerated.

### (2) Preparation of Transformants

The transformant of the invention may be obtained by introducing the recombinant vector of the invention into a host so that the gene of interest can be expressed. The host is not particularly limited as long as it can express the gene of the invention. Specific examples of hosts which may be used in the invention include *Escherichia* bacteria such as E. *coli; Bacillus* bacteria such as *Bacillus subtilis; Pseudomonas* bacteria such as *Pseudomonas putida;* yeasts such as *Saccharomyces cerevisiae*, *Schizosaccharomyces pombe*; animal cells and insect cells.

When a bacterium such as *E. coli* is used as a host, the recombinant vector of the invention is preferably not only capable of autonomous replication in the host but also composed of a promoter, a ribosome binding sequence, the gene of the invention and a transcription termination sequence. The vector may also contain a gene that controls the promoter.

Specific examples of *E. coli* strains which may be used in the invention include BL21 (DE3), JM109 and HB101. Specific examples of *Bacillus subtilis* strains which may be used in the invention include WB700 and LKS87.

As the promoter, any promoter may be used as long as it can direct the expression of the gene of the invention in a host such as *E*. *coli.* For example, an *E. coli-* or phage-derived promoter such as trp promoter, lac promoter, PL promoter or PR promoter, or an *E*. *coli*-infectious phage-derived promoter such as T7 promoter may be used. An artificially altered promoter such as tac promoter may also be used.

As a method for introducing the recombinant vector into a bacterium, any method of DNA transfer into bacteria may be used. For example, a method using calcium ions (Cohen, S.N. et al., Proc. Natl. Acad. Sci., USA, 69:2110-2114 (1972)) or electroporation may be used.

When yeast is used as the host, *Saccharomyces cerevisiae, Schizosaccharomyces pombe* or *Pichia pastoris* may be used, for example. A promoter which may be used in this case is not particularly limited. Any promoter may be used as long as it can direct the expression of the gene of the invention in yeast. For example, GAL1 promoter, GAL10 promoter, heat shock protein promoter, MF α 1 promoter, PH05 promoter, PGK promoter, GAP promoter, ADH promoter, AOX1 promoter or the like may be enumerated. As a method of introducing the recombinant vector into the yeast, any method of DNA transfer into yeast may be used. For example, electroporation (Becker, D.M., Methods Enzymol., 194:182-187 (1990)), the spheroplast method (Hinnen, A. et al., Proc. Natl. Acad. Sci., USA, 75:1929-1933 (1978)), the lithium acetate method (Itoh, H., J. Bacteriol., 153:163-168 (1983)) or the like may be enumerated.

When an animal cell is used as the host, simian COS-7 or Vero cells; Chinese hamster ovary cells (CHO cells); mouse L cells; rat GH3, PC12 or NG108-15 cells; human FL, HEK293, HeLa or Jurkat cells; or the like may be used. As a promoter, SR α promoter, SV40 promoter, LTR promoter, β -actin promoter or the like may be used. The early gene promoter of human cytomegalovirus may also be used. As a method of introducing the recombinant vector into the animal cell, electroporation, the calcium phosphate method, or lipofection may be used, for example.

When an insect cell is used as the host, Sf9 cells, Sf21 cells or the like may be used. As a method for introducing the recombinant vector into the insect cell, the calcium phosphate method, lipofection, or electroporation may be used, for example.

### 4. Production of Truncated MAGE-3

The truncated MAGE-3 of the invention can be obtained by culturing the above-described transformant and recovering the protein from the resultant culture. The term "culture" means any of the following materials: culture supernatant, cultured cells or microorganisms, or disrupted products from cultured cells or microorganisms. The cultivation of the transformant of the invention is carried out in accordance with conventional methods commonly used for culturing hosts.

As a medium to culture the transformant obtained from a microorganism host such as *E. coli* or yeast, either a natural or synthetic medium may be used as long as it contains carbon sources, nitrogen sources and inorganic salts assimilable by the microorganism and is capable of effective cultivation of the transformant.

As carbon sources, carbohydrates such as glucose, fructose, sucrose, starch; organic acids such as acetic acid, propionic acid; and alcohols such as ethanol and propanol may be used. As nitrogen sources, ammonia; ammonium salts of inorganic or organic acids such as ammonium chloride, ammonium sulfate, ammonium acetate, ammonium phosphate; other nitrogen-containing compounds; Peptone; meat extract; corn steep liquor and the like may be used. As inorganic substances, potassium dihydrogen phosphate, dipotassium hydrogen phosphate, magnesium phosphate, magnesium sulfate, sodium chloride, iron(II) sulfate, manganese sulfate, copper sulfate, calcium carbonate and the like may be used.

Usually, the cultivation is carried out under aerobic conditions (such as shaking culture or aeration agitation culture) at 37 °C for 6 to 24 hrs. During the cultivation, the pH is maintained at 6.5 to 7,5. The pH adjustment is carried out using an inorganic or organic acid, an alkali solution or the like. During the cultivation, an antibiotic such as ampicillin or tetracycline may be added to the medium if necessary.

When a microorganism transformed with an expression vector containing an inducible promoter is cultured, an inducer may be added to the medium if necessary. For example, when a microorganism transformed with an expression vector containing a promoter which is inducible by isopropyl-*β* -D-thiogalactoside (IPTG) is cultured, IPTG may be added to the medium. When a microorganism transformed with an expression vector containing trp promoter which is inducible by indoleacetic acid (IAA) is cultured, IAA may be added to the medium.

As a medium to culture a transformant obtained from an animal cell as a host, commonly used RPMI1640 medium or DMEM medium, or one of these media supplemented with fetal bovine serum, etc. may be used. Usually, the cultivation is carried out in the presence 5% CO₂ at 37 °C for 1 to 30 days. During the cultivation, an antibiotic such as kanamycin or penicillin may be added to the medium if necessary.

After the cultivation, the truncated MAGE-3 of the invention is recovered by disrupting the microorganisms or cells by such methods as sonication, repeated freezing & thawing, or treatment with a homogenizer, if the protein is produced within the microorganisms or cells. If the truncated MAGE-3 of the invention is produced outside the microorganisms or cells, the culture fluid is used as it is or subjected to centrifugation to remove the microorganisms or cells. Thereafter, the resultant supernatant is subjected to conventional biochemical techniques used for isolating/purifying proteins. These techniques include ammonium sulfate precipitation, gel chromatography, ion exchange chromatography and affinity chromatography, and may be used independently or in an appropriate combination. Thus, the protein of the invention can be isolated and purified from the above-mentioned culture.

### 5. Cell Death Inhibitors

MAGE-3 and truncated MAGE-3 react specifically with caspase-12 or the precursor thereof to thereby inhibit the processing from the precursor to the mature enzyme. Therefore, MAGE-3 or truncated MAGE-3 may be used as a cell death inhibitor. Also, MAGE-3 or truncated MAGE-3 may be used for treating or preventing diseases associated with cell death (cell death-related diseases). Furthermore, *MAGE-3* or truncated *MAGE-3* is useful as an agent for gene therapy. In addition, by determining the amount of MAGE-3 contained in cells, it is possible to examine the ability of the cells or tissue to resist apoptosis.

Specific examples of cell death-related diseases include Alzheimer's disease, neurodegenerative diseases, autoimmune diseases, amyotrophy and organ disorders. These diseases may be treated with the therapeutic agent of the invention regardless of whether they have been developed independently or in a form of complication. A combination of the above-mentioned disease(s) with other disease(s) is also included in the complication.

The therapeutic agent or agent for gene therapy of the invention can be administered orally or parenterally and systemically or locally.

When the protein or the gene of the invention is used as a prophylactic or therapeutic agent (including agent for gene therapy) for cell death-related diseases, the target is not particularly limited. For example, the agent of the invention may be used for a specific purpose of treating or preventing a cell death-related disease developing in tissues in the nervous system (such as the brain, spinal cord), the vascular system (such as the artery, vein, heart), the respiratory system (such as the trachea, lung), the digestive system (such as the salivary gland, stomach, intestine, liver, pancreas), the lymph system (such as the lymph node, spleen, thymus), the uninary system (such as the kidney), or the reproductive system (such as the testis, ovary, uterus). Such a disease may be an independent disease, or may be complicated with other cell death-related disease, or may be complicated with even a disease other than cell death-related diseases.

When the therapeutic agent of the invention is administered orally, the agent may be prepared into any of the formulations such as tablets, capsules, granules, powder, pills, troches, internal liquid agents, suspensions, emulsions or syrups. Alternatively, the therapeutic agent may be prepared into a dry product which is re-dissolved just before use. When the therapeutic agent of the invention is administered parenterally, the agent may be formulated into intravenous injections (including drops), intramuscular injections, intraperitoneal injections, subcutaneous injections, suppositories, or the like. Injections are supplied in the form of unit dosage ampules or multi-dosage containers.

These formulations may be prepared by conventional methods using appropriate excipients, fillers, binders, wetting agents, disintegrating agents, lubricating agents, surfactants, dispersants, buffers, preservatives, dissolution aids, antiseptics, flavoring/perfuming agents, analgesics, stabilizers, isotonic agents, etc. conventionally used in pharmaceutical preparations.

Each of the above-described formulations may contain pharmaceutically acceptable carriers or additives. Specific examples of such carriers or additives include water, pharmaceutically acceptable organic solvents, collagen, polyvinyl alcohol, polyvinylpyrrolidone, carboxyvinyl polymers, sodium alginate, water-soluble dextran, sodium carboxymethyl starch, pectin, xanthan gum, gum arabic, casein, gelatin, agar, glycerol, propylene glycol, polyethylene glycol, vaseline, paraffin, stearyl alcohol, stearic acid, human serum albumin, mannitol, sorbitol and lactose. One or a plurality of these additives are selected or combined appropriately depending of the form of the preparation.

The dosage levels of the therapeutic agent of the invention will vary depending on such factors as the age of the subject, the route of administration and the number of times of administration, and may be varied in a wide range. When an effective amount of the protein of the invention is administered in combination with an appropriate diluent and a pharmaceutically acceptable carrier, the effective amount of the protein may be in the range from 0.0001 to 1000 mg/body weight per administration. The therapeutic agent may be administered once a day or in several dosages per day.

When the gene of the invention is used as an agent for gene therapy, the gene of the invention may be directly administered by injection. Alternatively, a vector incorporating the gene of the invention may be administered. Specific examples of a suitable vector for this purpose include an adenovirus vector, adeno-associated virus vector, herpes virus vector, vaccinia virus vector and retrovirus vector. By using such a virus vector, the gene of the invention can be administered efficiently. Alternatively, the gene of the invention may be enclosed in phospholipid vesicles such as liposomes, and the resultant liposomes may be administered to the subject. Since liposomes are closed vesicles containing a biodegradable material, the gene of the invention and liposomes are mixed so that the gene is retained in the internal aqueous layer and the lipid bilayer of the liposomes (a liposome-gene complex is formed). Subsequently, when this complex is cultured with cells, the gene in the complex is taken into the cells (lipofection). Then, the resultant cells may be administered by the methods as described below.

As a method of administration of the agent for gene therapy of the invention, local administration to the central nervous system (such as the brain, spiral cord), the vascular system (such as the artery, vein, heart), the respiratory system (such as the trachea, lung), the digestive system (such as the salivary gland, stomach, intestine, liver, pancreas), the lymph system (such as the lymph node, spleen, thymus), the uninary system (such as the kidney), the reproductive system (such as the testis, ovary, uterus), etc. may be performed in addition to conventional systemic administration such as intravenous or intra-arterial administration. Further, an administration method combined with catheter techniques and surgical operations may also be employed.

The dosage levels of the agent for gene therapy of the invention vary depending on such factors as the age, sex and conditions of the subject, the route of administration, the number of times of administration, and the type of the formulation. Usually, it is appropriate to administer the gene of the invention in an amount of 0.1-100 mg/adult body per day.

### 6. Detection of Anti-Apoptotic Activity

The present invention is applicable to the detection of anti-apoptotic activity in cells or tissues. Briefly, those cells or tissues that have been shown expressing MAGE-3 (including truncated form thereof ) highly as a result of determination of MAGE-3 are judged to have acquired anti-apoptotic activity. The detection and quantitative determination of MAGE-3 or truncated form thereof may be performed by reacting it with an antibody (polyclonal or monoclonal) which specifically recognizes MAGE-3 or truncated form thereof. An extract prepared from a cell or tissue is spotted on a membrane such as nitrocellulose membrane to thereby prepare a dot blot, or an extract is electrophoresed and transferred onto a nitrocellulose membrane to thereby prepare a Western blot. Then, anti-MAGE-3 antibody and a secondary antibody are reacted with the blot in order to thereby detect MAGE-3. Alternatively, an extract is directly subjected to such technique as ELISA (enzyme-linked immunosorbent assay) to quantitatively determine MAGE-3. It is also possible to immunologically stain cells or tissues with anti-MAGE-3 antibody directly to thereby detect those cells or tissues that have acquired anti-apoptotic activity.

Furthermore, it is also possible to determine anti-apoptotic activity in tissues or cells by detecting mRNA encoding MAGE-3 or a truncated form thereof in the tissues or cells. The detection of mRNA can be performed by applying *in situ* hybridization or *in situ* RT-PCR (reverse transcription/polymerase chain reaction) to tissues or by applying Northern blotting or RT-PCR to nucleic acids extracted from tissues or cells.

### PREFERRED EMBODIMENTS OF THE INVENTION

Hereinbelow, the present invention will be described more specifically with reference to the following Examples. However, the technical scope of the present invention is not limited to these Examples. The nucleic acid-related reagents and enzymes used in these examples were purchased from Takara Biochemicals (Japan) or New England Biolabs (U.S.A.). The medium materials for culturing E. *coli* were purchased from Difco (U.S.A.). The medium materials for culturing mammalian cells were purchased from Gibco-BRL (U.S.A.). Unless otherwise indicated, other reagents were those manufactured by Sigma-Aldrich (U.S.A.).

### EXAMPLE 1

### Cloning of MAGE-3

A PCR (polymerase chain reaction) was performed using a human testis cDNA library (Clontech, U.S.A.) as a template to thereby obtain the coding region of MAGE-3 protein for use in a large scale expression experiment. An outline of the procedures is as described below. Not only in this Example but also in the subsequent Examples, general procedures for handling DNA and RNA were in accordance with the methods described by Sambrook et al. (Sambrook, J., Fritsch, E.F., & Maniatis, T., Molecular Cloning: A Laboratory Manual, 2nd Ed., Cold Spring Harbor Laboratory Press, Cold Spring Harbor, New York, USA (1989)).

Although high expression of MAGE-3 is observed in many cancer cells including melanoma cells, the expression of MAGE-3 in normal cells is limited to the testis (Van Pel, A. et al., Immunol. Rev. 145, 229-250 (1995)). The primers used in the PCR (i.e., NTOP77, NBOT68, NTOP79 and NBOT65) had the sequences as described below. In order to facilitate the subsequent cloning, a 5' primer (NTOP79) contained a sequence GATATC which can be cut by restriction enzyme *Eco*RV, and a 3' primer (NBOT65) contained a sequence CTCGAG which can be cut by restriction enzyme *Xho*I.

NTOP77: GCCCAGCTCCTGCCCACACT (SEQ ID NO: 5)
NBOT68: GGATGCGGCCCCGGAAGGT (SEQ ID NO: 6)
NTOP79: CTCGATATCGCACCATGCCTCTTGAGCAGAGG (SEQ ID NO: 7)
NBOT65: CTCCTCGAGTCACTCTTCCCCCTCTCT (SEQ ID NO: 8)

A set of DNA polymerase and a reaction buffer for PCR (Expanded High Fidelity PCR System) was purchased from Boehringer Mannheim (Germany). Reaction conditions of the PCR were as follows. Briefly, the 1st round PCR was performed with 1 ng of testis cDNA as a template using primers NTOP77 and NBOT68. This 1st round reaction was carried out 40 cycles, one cycle consisting of denaturing (at 94°C for 1 min), primer annealing (at 59°C for 1 min) and DNA extension (at 72°C for 1 min). One thousandth (1/1000) of the resultant PCR product was supplied to the 2nd round PCR. Using primers NTOP79 and NBOT65, the 2nd round PCR was performed 35 cycles, one cycle consisting of denaturing (at 94°C for 1 min), primer annealing (at 50°C for 1 min) and DNA extension (at 72°C for 1 min).

As a result of these PCR reactions, a DNA fragment of approximately 900 bp was specifically amplified. After cutting both ends of this fragment with restriction enzymes *EcoRV* and XhoI (both from Takara Biochemicals), the fragment was purified by agarose gel electrophoresis followed by DNA extraction (using Geneclean kit manufactured by Bio101, U.S.A.). The purified DNA fragment was inserted between the *Eco*RV and *Xho*I sites of a common vector pBluescript SK(-) to thereby obtain plasmid pNB178.

### EXAMPLE 2

### Large Scale Production of MAGE-3

MAGE-3 was produced in a large scale using *E*. *coli.*

First, the *MAGE-3* cloned in pNB178 was amplified by PCR. A sequence which can be cut by restriction enzyme *Nhe*I was added to the 5' primer, and a sequence which can be cut by restriction enzyme *Xho*I was added to the 3' primer. The reaction was performed 25 cycles, one cycle consisting of denaturing (at 94°C for 1 min), primer annealing (at 51°C for 1 min) and DNA extension (at 72°C for 2 min).

The amplified DNA fragment was purified in the same manner as described in Example 1, and then inserted between the *Nhe*I and *Xho*I sites of an *E*. *coli* expression plasmid vector pRSET-A (Invitrogen, U.S.A.) to thereby obtain plasmid pNB202. The use of this pREST-A vector adds a tag sequence containing His-His-His-His-His-His (Met-Arg-Gly-Ser-His-His-His-His-His-His-Gly-Met-Ala-Ser: SEQ ID NO: 9) to the 5' end of the cloned gene. This enables affinity purification of the expressed protein using an affinity resin with nickel ions (e.g., Probond; Invitorogen).

*E. coli* BL21 (DE3) pLysS strain was transformed with pNB202, The resultant transformants were cultured in LB medium containing ampicillin (100 µ g/ml) and chloramphenicol (34 µ g/ml) (Sambrook, J., Fritsch, E.F., & Maniatis, T., Molecular Cloning: A Laboratory Manual, 2nd Ed., Cold Spring Harbor Laboratory Press, Cold Spring Harbor, New York, USA (1989)). The expression of the *MAGE-3* was induced by adding 0.2 mM isopropyl-*β*-D-thiogalactoside (IPTG) to the medium containing transformants at the logarithmic growth phase. The MAGE-3 protein produced by forced expression was purified using Probond affinity resin (Invitrogen) according to the protocol of Novagen (U.S.A.).

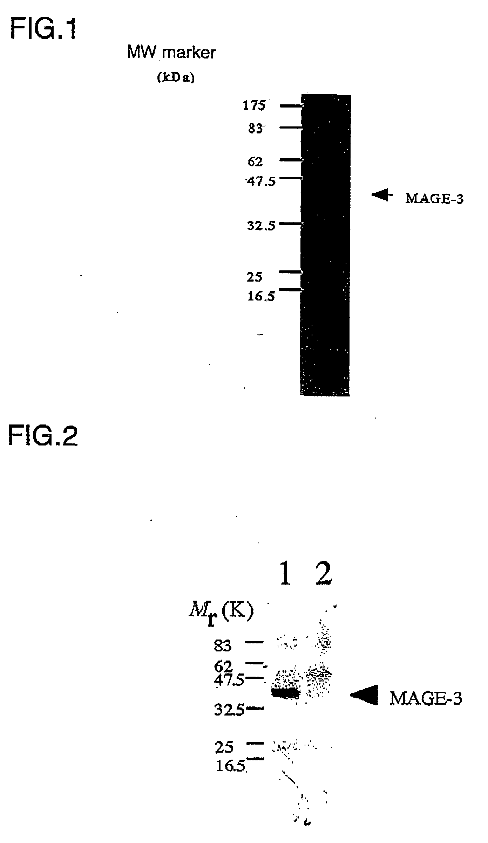

As a result, approximately 100 µg of purified MAGE-3 was obtained from 1 liter of the culture liquid. It was confirmed by SDS-polyacrylamide gel electrophoresis that the purity of the thus purified MAGE-3 was high (Fig. 1).

### EXAMPLE 3

### Binding Experiment between MAGE-3 and Pro-Caspase-12

Pro-caspase-12 and MAGE-3 protein were over-expressed in COS-1 cells. Then, the binding of them in an extract from the COS-1 cells was examined by the immunoprecipitation technique.

An anti-MAGE-3 antibody to be used in the detection of immunoprecipitate was prepared by immunizing rabbits. Briefly, a peptide representing a C-terminal sequence of MAGE-3 (CHISYPPLHEWVLREGEE ; SEQ ID NO: 10; Tana Laboratories, U.S.A.) was linked to a carrier protein (activated hemocyanin; Pierce, U.S.A.) and injected into rabbits together with an adjuvant. The "C" at the amino terminus of the above peptide was added artificially so that the peptide binds to the carrier protein and an affinity resin to be used. On the other hand, a peptide of the same sequence as described above was coupled to activated FMP-Cellulofine resin (Seikagaku Corp., Japan) to prepare a peptide column, which was used in the subsequent affinity purification. The anti-serum obtained from the immunized rabbit was applied to the peptide column to thereby allow the specific antibody to bind to the peptide. The specific antibody was eluted from the column with a glycine solution (pH 2.6). The pH of the effluent was raised to a neutral pH value by adding thereto 1 M Tris. The specific antibody contained therein was used in the subsequent experiment.

A cDNA encoding pro-caspase-12 and a cDNA encoding MAGE-3 were cloned separately into a mammalian cell expression plasmid pcDNA3.1(-) (Invitrogen, U.S.A.) and transferred into COS-1 cells by transient co-transfection. For pro-caspase-12, a genetically engineered gene was used which would add a FLAG sequence (Hopp, T.P. et al., Bio/Technol. 6, 1204-1210 (1988)) to the amino terminal of the expressed product. For transferring these genes into COS-1 cells, Superfect Transfection Kit (Qiagen, Germany) was used.

Two days after the transfection, COS-1 cells were harvested to prepare a cell extract. Using an anti-FLAG affinity gel (Sigma-Aldrich, U.S.A.), FLAG-added caspase-12 was selectively immunoprecipitated from the cell extract. The detection of proteins was carried out by Western blotting (Harlow, E. & Lane, D., Antibodies: A Laboratory Manual, Cold Spring Harbor Laboratory Press, Cold Spring Harbor, New York, U.S.A. (1988)) using ECL-plus kit (Amersham-Pharmacia).

The results are shown in Fig. 2. In Fig. 2, lane 1 shows that MAGE-3 co-exists in the immunoprecipitate. This occurred because MAGE-3 had bound to the pro-caspase-12 precipitated by the anti-FLAG affinity gel. When MAGE-3 alone was over-expressed in COS-1 cells as a control experiment, MAGE-3 could not be immunoprecipitated by the anti-FLAG affinity gel (lane 2).

Thus, it was revealed that MAGE-3 protein and pro-caspase-12 form a stable complex in cultured mammalian cells.

### EXAMPLE 4

### Examination of the MAGE-3-Binding Region within Caspase-12 and its Specificity

The MAGE-3-binding region within caspase-12 was examined according to the yeast two hybrid method developed by R. Brent et al. in the U.S. (Gyuris, J. et al., Cell 75, 791-803 (1993)). According to this method, the binding of two proteins can be detected when a yeast strain containing genes of these two proteins exhibits β-galactosidase activity.

Briefly, a cDNA encoding MAGE-3 was cloned into vector pJG4-5 to thereby create a fusion gene composed of *MAGE-3* and a gene encoding the gene expression-activating protein B42. The plasmid containing this fusion gene was designated pNB321.

A cDNA fragment corresponding to p10 subunit contained in mature caspase-12 (Thornberry, N.A. & Lazebnik, Y., Science 281, 1312-1316 (1998)) was cloned into vector pEG202 to thereby create a fusion gene composed of *p10* and the DMA-binding region of LexA. The plasmid containing this fusion gene was designated pNB316.

These plasmids pNB321 and pNB316 as well as plasmid pSH18-34 containing a β-galactosidase reporter gene were introduced into a budding yeast strain EGY48 for assay purpose to thereby create NMY307 strain. NMY307 was cultured in a synthetic medium [0.67% yeast nitrogen source (Difco, U.S.A.); histidine-, tryptophan- and uracil-free amino acids/nucleic acids mixture (Bio-101, U.S.A.), 2% galactose]. Then, β-galactosidase activity in NMY307 was examined according to the method of Gyuris et al. (Gyuris et al., Cell 75, 791-803 (1993)).

On the other hand, β-galactosidase activity was examined in the same manner on those yeast strains containing gene fragments encoding p10 regions of other caspases (i.e., caspase-1, -2, -3 and -8) (individually cloned into pEG202) and *MAGE-3* (cloned into pJG4-5).

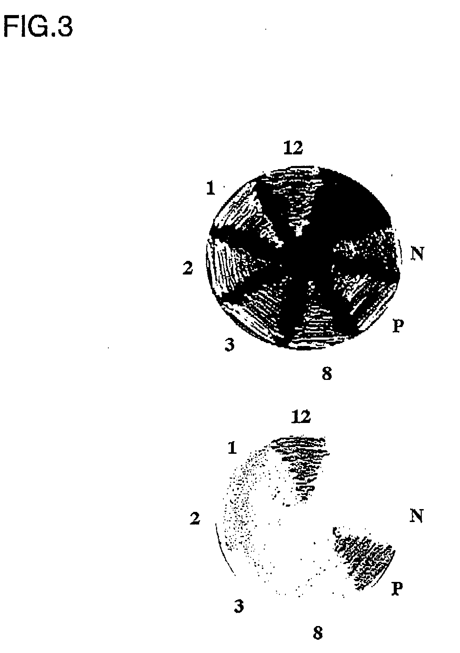

The results are shown in Fig. 3. In Fig. 3, "1", "2", "3", "8" and "12" represent yeast cells containing gene fragments encoding p10 region of caspase-1, -2, -3, -8 and-12, respectively, together with *MAGE-3.* "P" represents a positive control strain prepared by introducing a gene encoding an active transcription factor (cloned in plasmid pSH17-4) and a *β*-galactosidase reporter gene (cloned in plasmid pSH18-34) into EGY48. "N" represents a negative control strain prepared by introducing a gene fragment encoding p10 region of caspase-12 and a β -galactosidase reporter gene into EGY48.

It was shown that yeast cells in which *β* -galactosidase activity is occurring generate a colored substance from the substrate X-Gal (5-bromo-4-chloro-3-indolyl-β-D-thiogalactoside) ("12" in the lower panel). This means that the p10 region of mature caspase-12 has bound to MAGE-3 protein. This β -galactosidase activity was almost equal to that observed in the positive control strain ("P" in the lower panel) in intensity. On the other hand, color development was observed little in the negative control strain ("N" in the lower panel). Furthermore, the color development in any of the other caspases tested (i.e., caspase-1, -2, -3 and -8) was as little as in the negative control strain "N" (Fig. 3, the lower panel). Thus, the binding of these caspases to MAGE-3 was not detected.

Consequently, it was revealed that p10 region located on the C-terminal side in cascape-12 (which becomes an approximately 10 kDa subunit in mature caspase-12) is necessary for the binding of caspase-12 to MAGE-3, and that the binding of MAGE-3 to caspase-12 is specific.

### EXAMPLE 5

### Binding Experiment between Caspase-12 and Truncated Forms of MAGE-3

Factors which can bind to the p10 region of pro-caspase-12 were searched for using a HeLa cell cDNA library.

A gene fragment encoding the p10 region of pro-caspase-12 was cloned into pEG202 and subjected to the interaction trapping technique developed by R. Brent et al. (Gyuris, J. et al., Cell 75, 791-803 (1993)). All of the resultant binding proteins were truncated forms of MAGE-3. These truncated forms were as described below. The shortest truncated form (Truncated Form 1) consists of the amino acid sequence spanning from the Gly residue at position 94 to the Glu residue at the C-terminus of the full-length MAGE-3 protein (represented by SEQ ID NO: 2).
- Truncated Form 1:: a MAGE-3 protein consisting of the amino acid sequence of SEQ ID NO: 2 from which amino acids 1-93 are deleted
- Truncated Form 2:: a MAGE-3 protein consisting of the amino acid sequence of SEQ ID NO: 2 from which amino acids 1-90 are deleted
- Truncated Form 3:: a MAGE-3 protein consisting of the amino acid sequence of SEQ ID NO: 2 from which amino acids 1-88 are deleted
- Truncated Form 4:: a MAGE-3 protein consisting of the amino acid sequence of SEQ ID NO: 2 from which amino acids 1-81 are deleted

For each of these truncated forms, its binding to caspase-12 was examined in accordance with the method of Gyuris et al. mentioned above. Briefly, a gene fragment encoding p10 region of pro-caspase-12, a gene fragment encoding a truncated MAGE-3 and a lacZ reporter gene were transformed into a budding yeast strain EGY48. The resultant transformant was cultured on synthetic galactose medium plates. Colonies formed on the plate were transferred onto a nylon membrane (Hybond-N⁺; Amersham-Pharmacia) and then dipped in liquid nitrogen to disrupt yeast cells. The nylon membrane on which disrupted cells were present was dipped in an X-Gal-containing phosphate buffer (Gyuris, J. et al., Cell 75, 791-803 (1993)) and retained at 30°C for 4-6 hr, Then, it was confirmed that a reaction generating blue color occurred on the surface of the membrane by the enzyme activity of the β -galactosidase produced when the lacZ gene in yeast cells was expressed as a result of the binding of p10 region of pro-caspase-12 to the truncated MAGE-3.

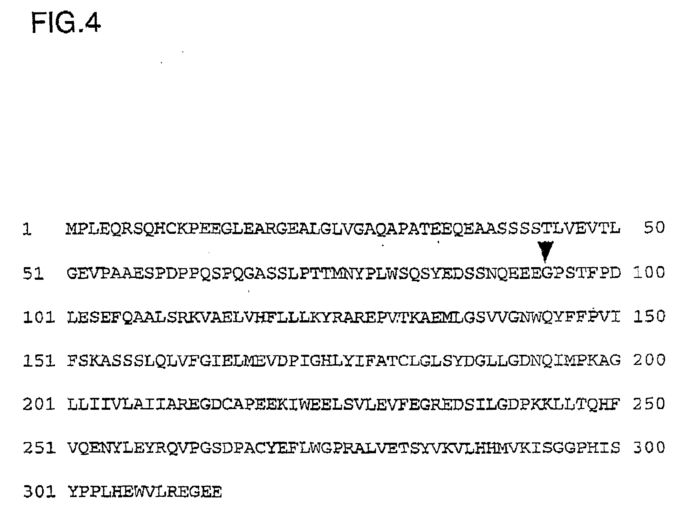

All of the truncated forms presented a strong color reaction. Consequently, it was shown that even the partial-length polypeptide of MAGE-3 spanning from Gly⁹⁴ to Glu³¹⁴ (C-terminus) [the region spanning from "▼" mark (position 94) to the C-terminus (position 314) in Fig. 4] has the ability to bind to caspase-12.

### EXAMPLE 6

### Binding Experiment between MAGE-3 Protein and Caspase-12 (Binding of mature caspase-12)

In this Example, to what extent the precursor and the active form of caspase-12 bind to MAGE-3 protein, respectively, was examined by GST-fusion protein binding experiments.

The mature caspase-12 (hereinafter, frequently referred to as "p30") used in these experiments was prepared by removing from pro-caspase-12 the N-terminal pro-domain sequence that is not included in mature (active) caspase-12 and then adding His-His-His-His-His-His sequence to the N-terminal. A cDNA fragment encoding this p30 protein was cloned into an *E*. *coli* expression plasmid vector pRSET-A (Invitrogen). When p30 is expressed in *E*. *coli* in a large scale, this protein is converted into the mature form by autoprocessing.

On the other hand, the immature caspase-12 used in these experiments was prepared as follows. Briefly, in order to stabilize immature caspase-12 (i.e., to prevent its autoprocessing), the Cys residue located in the active site of caspase-12 was replaced with a Ser residue to thereby create a mutant p30 (designated "p30C/S"). This mutant was expressed separately in *E*. *coli* in a large scale. The cultivation of *E*. *coli* and the induced synthesis of p30 proteins were carried out in accordance with the protocols of Invitrogen. Both p30 and p30C/S were purified by nickel column affinity chromatography (Probond Affinity Resin; Invitrogen) utilizing the N-terminal 6x(His) sequence.

For the purpose of easy recovery of caspase-12-bound products, a cDNA encoding a fusion protein composed of MAGE-3 and glutathione-S-transferase (GST) fused to its N-terminal was prepared (pNB341). Briefly, a cDNA encoding MAGE-3 was cloned into an E. *coli* expression plasmid vector pGEX-4T-3 (Amersham-Pharmacia; Sweden) so that the reading frame of the former coincides with the reading frame of the latter, to thereby construct a fusion gene. After the introduction of pNB341 into *E*. *coli* XL-2 Blue MRF', cultivation of the cells and induced synthesis of the GST-MAGE-3 fusion protein were carried out according to the protocol of Amersham-Pharmacia. The GST-MAGE-3 fusion protein was purified from an extract of the *E*. *coli* cells using a glutathione resin (Glutathione Sepharose 4B; Amersham-Pharmacia). The purification was performed according to the protocol of Amersham-Pharmacia.

The purified GST-MAGE-3 protein was mixed with p30 (mature caspase-12) or p30C/S (immature caspase-12) and then recovered from the solution with the glutathione resin. The proteins co-precipitated with the GST-MAGE-3 protein were analyzed by Western blotting.

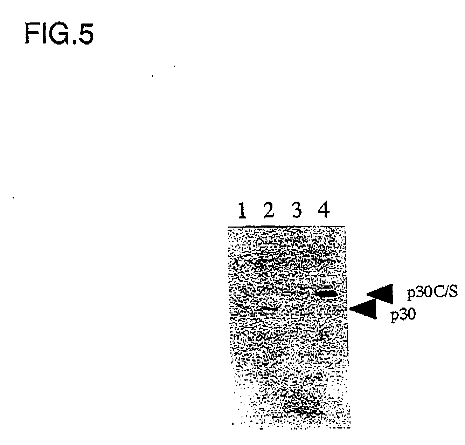

The results are shown in Fig. 5. Legends on individual lanes in this Figure are as follows.
Lane 1: control experiment in which mature caspase-12 and GST were mixed (MAGE-3 was not contained)
Lane 2: mature caspase-12 recovered together with GST-MAGE-3 as a precipitate
Lane 3: control experiment in which pro-caspase-12 and GST were mixed (MAGE-3 was not contained)
Lane 4: immature caspase-12 recovered together with GST-MAGE-3 as a precipitate

As seen from Fig. 5, although both p30 (mature caspase-12) and p30C/S (immature caspase-12) were recognized in co-precipitates, p30C/S was recognized more than p30 clearly. Accordingly, it was found out that immature caspase-12 binds to GST-MAGE-3 protein more efficiently than mature caspase-12, though both of them bind thereto.

### EXAMPLE 7

### Inhibition of Caspase Activation by MAGE-3

In this Example, a pro-caspase-12 (48 kDa) synthesized *in vitro* was cleaved by mature caspase-12 to thereby reproduce the *in vivo* activation *in vitro.*

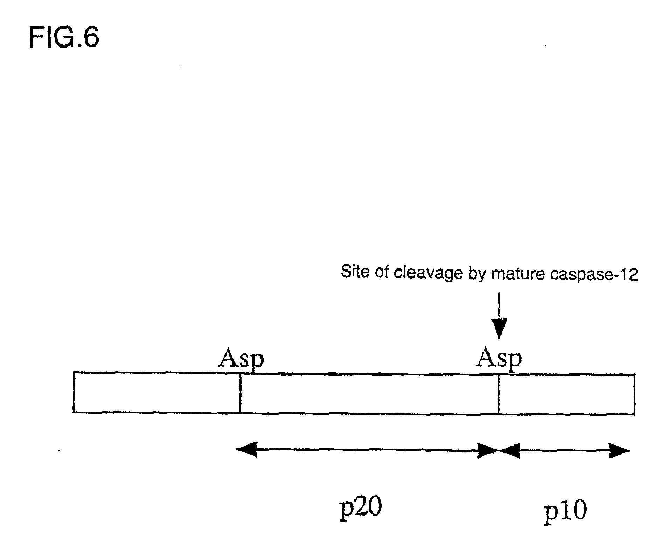

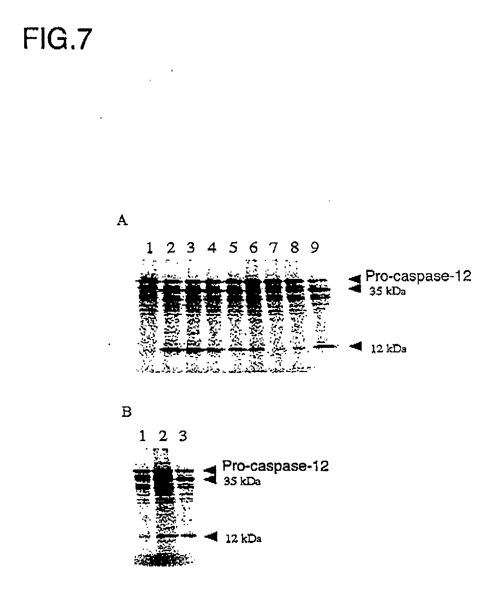

The site of cleavage of pro-caspase-12 by mature caspase-12 is specific. This cleavage site is a specific Asp residue located on the border between the about 20 kDa and about 10 kDa subunits (called p20 and p10, respectively) contained in mature caspase-12 (Fig. 6; lane 2, Fig. 7). It is possible to inhibit this specific cleavage by adding MAGE-3 protein to the cleavage reaction. The experiment was carried out as described below.

Briefly, pro-caspase-12 was synthesized using a cDNA coding therefor and an in *vitro* protein synthesis kit (TNT *in vitro* transcription/translation kit; Promega, U.S.A.). ³⁵S-labeled methionine (Amersham-Pharmacia) was added to the synthesis reaction so that the resultant proenzyme was radioactively labeled.

The radioactively labeled pro-caspase-12, mature caspase 12 and MAGE-3 protein were mixed in a buffer containing 20 mM Tris-HCl (pH 7.0) and 10 mM dithiothreitol. Then, cleavage reaction was carried out at 37°C for 45 min. The reaction was terminated by adding thereto an equal volume of 2X electrophoresis sample buffer [100 mM Tris-HCl (pH 6.8), 2% sodium lauryl sulfate, 10% mercaptoethanol, 0.2% Bromophenol Blue, 20% glycerol] and heating the solution at 100°C for 4 min.

This reaction solution was developed by SDS-polyacrylamide gel (14%). The resultant gel was treated with a sensitizer (Ampify; -Amersham-Pharmacia), dried and subjected to autoradiography. The radioactive protein contained in the dried gel was detected using BAS2500 detection system (Fuji Film, Japan).

The results are shown in Fig. 7. Legends on individual lanes are as follows.
(1) Panel A
   Lane 1: Pro-caspase-12 synthesized *in vitro* in the presence of radioactive methionine
   Lanes 2-7: Pro-caspase-12 cleaved by mature caspase-12 (0.28 *µ* g). The cleavage reaction was carried out in the presence of MAGE-3 protein in the amount as indicated below:
      Lane 2 (0 µg), Lane 3 (0.3 µg), Lane 4 (1.5 µg), Lane 5 (3 µg), Lane 6 (6 µg), Lane 7 (12 µg).
   Lane 8: Pro-caspase-12 cleaved by mature caspase-12 (1.1 *µ* g). The cleavage reaction was carried out in the presence of 12 *µ* g of MAGE-3 protein.
   Lane 9: Pro-caspase-12 cleaved by mature caspase-12 (1.1 µg). A control sample. The cleavage reaction was carried out in the presence of 12 µ g of bovine serum albumin instead of MAGE-3 protein.
(2) Panel B
   Lane 1: A sample similar to that of lane 7, Panel A (i.e., twice as much substrate (pro-caspase-12) was added to the reaction mixture).
   Lane 2: A sample similar to that of lane 7, Panel A (i.e., the cleavage reaction was carried out in the presence of 24 *µ*g of MAGE-3).
   Lane 3: The same sample as that of lane 9, Panel A

Lanes 2 through 7 in Fig. 7A show the results of experiments in which gradually increasing amounts (0 to 12 *µ* g) of MAGE-3 protein was present in the reaction. Addition of 12 µg of MAGE-3 inhibited the cleavage reaction by caspase-12 almost completely. When 12 *µ*g of bovine serum albumin was added instead of MAGE-3 as a control experiment, no inhibitory effect was observed (lane 9, Fig. 7A). The inhibitory effect is resulted mainly from the binding of MAGE-3 to pro-caspase -12.

Furthermore, the addition of excessive mature caspase-12 (i.e., 1.1 *µ* g; 4 times the amount used in lanes 2-7, Fig. 7A) under the conditions in which cleavage was almost completely inhibited by 12 *µ* g of MAGE-3 did not restore cleavage (lane 8, Fig. 7A). On the other hand, when an excessive amount (i.e., 0.4 *µ* 1 of the *in vitro* tranlated protein solution; two times the amount used in lane 1, panel B and lanes 1 through 9, panel A) of substrate (pro-caspase-12) was added under similar conditions, specific cleavage could be detected again (lane 2, Fig. 7B).

These results revealed that caspase is activated by specific intramolecular cleavage. It was also revealed that MAGE-3 binds to pro-caspase-12 strongly to thereby inhibit the activation of caspase-12.

### EXAMPLE 8

### Anti-Apoptotic Effect of MAGE-3 in Cultured Cells

This Example was to confirm that high expression of MAGE-3 in cultured cells enhances the cells' resistance to endoplasmic reticulum (ER) stress-dependent apoptosis.

In order to examine the inhibition of caspase-12 activation by MAGE-3 at cell level, cell clones that express MAGE-3 highly were established. Then, the reaction of the cells to apoptotic stimuli was examined. Expression of MAGE-3 has not been detected in normal tissues except the testis (Van Pel, A. et al., Immunol. Rev, 145, 229-250 (1995)).

Briefly, plasmid pNB179 containing a cDNA encoding MAGE-3 was introduced into a mouse myoblast cell strain, C2C12, using Superfect Transfection kit. Since pNB179 contains the drug resistant gene *neo,* transformed cells containing this plasmid integrated into chromosomes were selectively grown in a medium containing the antibiotic Geneticin (Gibco-BRL). After 2-week selective culture, colonies of resistant cells formed on culture plates were picked up, cultured separately and then cloned. The amount of MAGE-3 expression in each clone was determined by Western blotting (using anti-MAGE-3 protein antibody). Thus, two clones (C2/MA13 and C2/MA21) highly expressing MAGE-3 were obtained.

Caspase-12 is activated by ER-dependent, apoptosis-inducing stimulus in cells (Nakagawa, T. et al., Nature 403, 98-103 (2000)). Then, the prevent inventor added thapsigargin, which is an inhibitor of ER-specific calcium ATPase, (Calbiochem, U.S.A.; Thastrup, O. et al., Proc. Natl. Acad. Sci. USA 87, 2466-2470 (1990)) to the culture medium of the above-described high expression clones at a concentration of 0.4 *µ* M (for 24 hr).

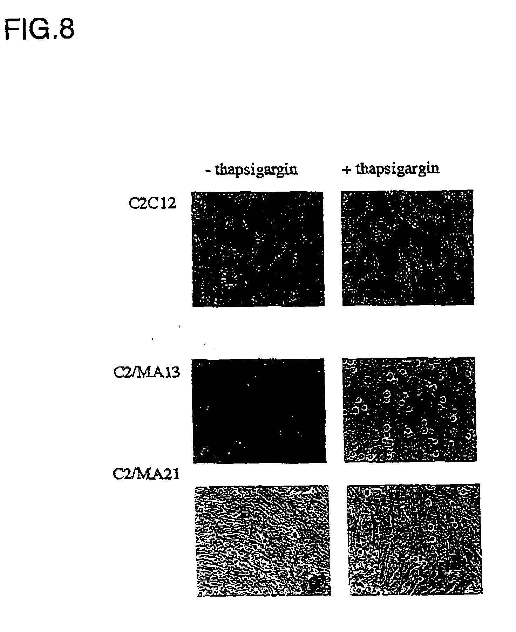

As a result, the intracellular calcium homeostasis was disrupted, which promoted activation of caspase-12. Consequently, apoptosis was induced in the cells. More specifically, when C2C12 cells (parent strain) were treated with 0.4 *µ* M thapsigargin for 24 hr, 50% or more of the cells died exhibiting apoptotic morphology ( right upper panel in Fig. 8).

On the other hand, when C2/MA13 and C2/MA21 clones were treated with thapsigargin in the same manner, the cells exhibited resistance. According to the microscopic observation, 90% or more of the cells of these clones retained normal morphology (right middle and right lower panels in Fig. 8). The left panels show the results of control experiments in which cells were treated with 0.1 % dimethyl sulfonic acid.

Subsequently, in order to quantitatively determine the resistance to thapsigargin treatment, the survival rate in each cell clone was calculated. The results of measurement using Cell Counting Kit-8 (Dojindo Co., Japan) revealed that while the survival rate in C2C12 decreased to 50% after the 24-hour thapsigargin treatment, the survival rates in C2/MA13 and C2/MA21 were retained at a high level (75%). Accordingly, it was shown that cells have resistance to ER-specific stress under high expression of MAGE-3. This means that MAGE-3 inhibits the development and progress of apoptosis.

All the publications, patents and patent applications cited in the present specification are incorporated herein by reference in their entireties.

### EFFECT OF THE INVENTION

According to the present invention, there are provided a protein which specifically binds to caspase-12 to thereby inhibit the activation thereof, and a cell death inhibitor containing the protein. This protein is useful as a drug for inhibiting cell death such as apoptosis.

### SEQUENCE LISTING FREE TEXT

SEQ ID NO: 5: synthetic DNA
SEQ ID NO: 6: synthetic DNA
SEQ ID NO: 7: synthetic DNA
SEQ ID NO: 8: synthetic DNA
SEQ ID NO: 9: synthetic peptide
SEQ ID NO: 10: synthetic peptide

## Claims

1. A recombinant protein selected from the group consisting of the following (a) and (b):
(a) a protein consisting of the amino acid sequence as shown in SEQ ID NO: 4
(b) a protein which consists of the amino acid sequence as shown in SEQ ID NO: 4 having deletion, substitution or addition of one or several amino acids and which inhibits the activation of caspase-12.

2. A gene coding for a recombinant protein selected from the group consisting of the following (a) and (b):
(a) a protein consisting of the amino acid sequence as shown in SEQ ID NO: 4
(b) a protein which consists of the amino acid sequence as shown in SEQ ID NO: 4 having deletion, substitution or addition of one or several amino acids and which inhibits the activation of caspase-12.

3. A gene consisting of a DNA selected from the group consisting of the following (c) and (d):
(c) a DNA consisting of the nucleotide sequence as shown in SEQ ID NO: 3
(d) a DNA which hybridizes to the nucleotide sequence as shown in SEQ ID NO: 3 under stringent conditions and which codes for a protein that inhibits the activation of caspase-12.

4. A recombinant vector comprising the gene of claim 2 or 3.

5. A transformant comprising the recombinant vector of claim 4.

6. A method of producing a protein which inhibits the activation of caspase-12, comprising culturing the transformant of claim 5 in a medium and recovering the protein from the resultant culture.

7. A complex formed by the binding of a MAGE-3 protein and/or a truncated form thereof to caspase-12 or the precursor thereof.

8. A cell death inhibitor comprising a MAGE-3 protein and/or a truncated form thereof as an active ingredient.

9. The cell death inhibitor of claim 8, wherein the MAGE-3 protein is a recombinant protein selected from the group consisting of the following (e) and (f):
(e) a protein consisting of the amino acid sequence as shown in SEQ ID NO: 2
(f) a protein which consists of the amino acid sequence as shown in SEQ ID NO: 2 having deletion, substitution or addition of one or several amino acids and which inhibits the activation of caspase-12.

10. The cell death inhibitor of claim 8, wherein the truncated form of a MAGE-3 protein is a recombinant protein selected from the group consisting of the following (a) and (b):
(a) a protein consisting of the amino acid sequence as shown in SEQ ID NO: 4
(b) a protein which consists of the amino acid sequence as shown in SEQ ID NO: 4 having deletion, substitution or addition of one or several amino acids and which inhibits the activation of caspase-12.

11. The cell death inhibitor of any one of claims 8 to 10, wherein the cell death is at least one selected from the group consisting of apoptosis, necrosis, scheduled cell death, programmed cell death and cell injury.

12. A therapeutic agent for cell death related diseases, comprising a MAGE-3 protein and/or a truncated form thereof as an active ingredient.

13. The therapeutic agent of claim 12, wherein the MAGE-3 protein is a recombinant protein selected from the group consisting of the following (e) and (f):
(e) a protein consisting of the amino acid sequence as shown in SEQ ID NO: 2
(f) a protein which consists of the amino acid sequence as shown in SEQ ID NO: 2 having deletion, substitution or addition of one or several amino acids and which inhibits the activation of caspase-12.

14. The therapeutic agent of claim 12, wherein the truncated form of a MAGE-3 protein is a recombinant protein selected from the group consisting of the following (a) and (b):
(a) a protein consisting of the amino acid sequence as shown in SEQ ID NO: 4
(b) a protein which consists of the amino acid sequence as shown in SEQ ID NO: 4 having deletion, substitution or addition of one or several amino acids and which inhibits the activation of caspase-12.

15. The therapeutic agent of any one of claims 12 to 14, wherein the cell death related disease is at least one selected from the group consisting of Alzheimer's disease, neurodegenerative diseases, autoimmune diseases, amyotrophy and organ disorders.

16. A method of inhibiting the activation of caspase-12, comprising binding a MAGE-3 protein or a truncated form thereof to caspase-12 or the precursor thereof.

17. A method of detecting anti-apoptotic activity in a tissue or cell, comprising treating said tissue or cell with an antibody which specifically recognizes a MAGE-3 protein or a truncated form thereof and detecting expression of said MAGE-3 protein or said truncated form in said tissue or cell, wherein detection of a high expression of said MAGE-3 protein or said truncated form thereof indicates that said tissue of cell has anti-apoptotic activity.

18. A method of detecting anti-apoptotic activity in a tissue or cell, comprising detecting the expression of an mRNA encoding a MAGE-3 protein or a truncated form thereof in said tissue or cell.
